# EUROPÄISCHE PATENTANMELDUNG

(11) **EP 3 289 856 A1**
(43) Veröffentlichungstag der Anmeldung: **07.03.2018**
(21) Anmeldenummer: 16187274.2
(22) Anmeldetag: 05.09.2016
(51) Int. Cl.: A01G 9/10, G01N 1/04, A01G 31/02

(54) **ANZUCHT- UND PROBENNAHME-VORRICHTUNG FÜR PFLANZEN**

(71) Anmelder: Deutsche Saatveredelung AG, 59557 Lippstadt (DE)
(72) Erfinder: Micic, Zeljko, 33154 Salzkotten (DE); Radtke, Simon, 59494 Soest (DE)
(74) Vertreter: Wickord, Wiro

(57) **Zusammenfassung**

Die Erfindung betrifft eine Probennahme-Vorrichtung für Pflanzen, wobei die Probennahme-Vorrichtung ein Unterteil (2) mit einer Mehrzahl von Probenbehältern (2.1), ein Oberteil (4) mit einer Mehrzahl von Anzuchtbehältern (4.1), ein Messer (6) und eine Schneidplatte (8) aufweist, wobei in jedem Anzuchtbehälter (4.1) eine Bodenöffnung (4.1.1) ausgebildet ist, die zu einer Probenbehälteröffnung eines der Probenbehälter (2.1) korrespondiert, und wobei das Messer (6) und die Schneidplatte (8) in einer Gebrauchslage der Probennahme-Vorrichtung derart zwischen dem Oberteil (4) und dem Unterteil (2) angeordnet sind, dass ein durch die Bodenöffnung (4.1.1) des Anzuchtbehälters (4.1) heraus- und durch die Probenbehälteröffnung in den Probenbehälter (2.1) hineinragendes Pflanzenteil mittels des Messers (6) und der Schneidplatte (8) durchtrennbar ist.

## Beschreibung

Die Erfindung betrifft eine Anzucht- und Probennahme-Vorrichtung für Pflanzen. Auf die erfindungsgemäße Vorrichtung wird im Weiteren der Einfachheit halber als Probenahme-Vorrichtung Bezug genommen.

Gattungsgemäße Probennahme-Vorrichtungen sind bereits bekannt und dienen unter anderem der phänotypischen Beschreibung und genetischen Untersuchung bei der Züchtung von Kulturpflanzen. Es handelt sich dabei beispielsweise um Zangen, mittels derer Gewebe aus Pflanzen ausgestanzt wird. Die ausgestanzten Pflanzenteile, beispielsweise Blattgrün, werden dann in Probenbehälter eingebracht und der weiteren labortechnischen Behandlung zugeführt.

Aufgabe der vorliegenden Erfindung ist es, eine Probennahme-Vorrichtung anzugeben, bei der der Arbeitsaufwand bei der Beschreibung und/oder Untersuchung und/oder Züchtung von Pflanzen weiter reduziert ist und die Züchtung von Kulturpflanzen effizienter zu gestalten.

Zur Lösung der Aufgabe ist die Erfindung in Verbindung mit dem Oberbegriff des Patentanspruchs 1 dadurch gekennzeichnet, dass die Probennahme-Vorrichtung ein Unterteil mit einer Mehrzahl von Probenbehältern, ein Oberteil mit einer Mehrzahl von Anzuchtbehältern, ein Messer und eine Schneidplatte aufweist, wobei in jedem Anzuchtbehälter eine Bodenöffnung ausgebildet ist, die zu einer Probenbehälteröffnung eines der Probenbehälter korrespondiert, und wobei das Messer und die Schneidplatte in einer Gebrauchslage der Probennahme-Vorrichtung derart zwischen dem Ober- und dem Unterteil angeordnet sind, dass ein durch die Bodenöffnung des Anzuchtbehälters heraus- und durch die Probenbehälteröffnung in den Probenbehälter hineinragendes Pflanzenteil mittels des Messers und der Schneidplatte durchtrennbar ist.

Der besondere Vorteil der Erfindung besteht darin, dass eine hohe Anzahl von Probennahmen mit viel weniger Handarbeit geleistet werden kann. Die Pflanzen können in den einzelnen Anzuchtbehältern herangezogen werden. Gegebenenfalls ist es von Vorteil, wenn die Anzuchtbehälter, beispielsweise in einer Vorkonfektion, bereits mit den hierfür erforderlichen Nährstoffen befüllt worden sind.

Während der Pflanzenentwicklung wachsen die Wurzeln der Pflanzen durch die Bodenöffnungen der Anzuchtbehälter heraus und durch die jeweils korrespondierende Probenbehälteröffnung in den entsprechenden Probenbehälter hinein. Die Probennahme erfolgt mittels des an der Schneidplatte entlanggleitenden Messers, wobei die Wurzeln der Pflanzen durchtrennt werden. Eine händische Zuordnung von manuell ausgestanzten Gewebeproben zu den einzelnen Probenbehältern ist nicht erforderlich. Damit sind auch fehlerhafte Zuordnungen sicher vermieden.

Eine besonders vorteilhafte Weiterbildung der erfindungsgemäßen Lehre sieht vor, dass das Messer und/oder die Schneidplatte als eine Lochplatte ausgebildet ist/sind. Auf diese Weise ist es möglich, beispielsweise jeder einzelnen Paarung von Anzuchtbehälter auf der einen Seite und Probenbehälter auf der anderen Seite ein individuelles Messer und/oder eine individuelle Schneidplatte zuzuordnen. Eine ungewünschte Durchmischung von Gewebeproben (Kreuzkontamination) ist dadurch wirksam verhindert.

Nach einer Weiterbildung können die Aufzuchtbehälter mit den Bodenöffnungen und die Probenbehälter mit den Probenbehälteröffnungen regelmäßig beabstandet und/oder matrixartig und/oder mosaikartig angeordnet sein. Beispielsweise können an dem Messer und der Schneidplatte in korrespondierender Weise regelmäßig beabstandet und/oder matrixartig und/oder mosaikartig Schneidlöcher nach Art von Durchgangsöffnungen vorgesehen sein.

Grundsätzlich ist die Schneidplatte nach Art, Material, Form, Dimensionierung und Anordnung in weiten geeigneten Grenzen frei wählbar. Zweckmäßigerweise ist die Schneidplatte als ein Deckel für das Unterteil ausgebildet.

Nach einer Weiterbildung der Erfindung ist das Messer flächig an der Schneidplatte angelegt und/oder relativ zu derselben längsverschiebbar gehalten. Das Messer und die Schneidplatte können zu diesem Zweck einander zugewandte, jedenfalls abschnittsweise eben ausgebildete Anlageflächen vorsehen.

Eine vorteilhafte Weiterbildung der erfindungsgemäßen Lehre sieht vor, dass eine dem Messer zugewandte Fläche des Unterteils und/oder eine dem Messer zugewandte Fläche des Oberteils als eine Messerführung ausgebildet ist/sind. Hierdurch ist das Messer zwischen der Schneidplatte auf der einen Seite und der auf die vorgenannte Weise ausgebildeten Messerführung auf der anderen Seite sicher geführt. Entsprechend ist die Schnittqualität verbessert.

Grundsätzlich ist die Art der mechanischen Verbindung zwischen den einzelnen Bauteilen der erfindungsgemäßen Probennahme-Vorrichtung in weiten geeigneten Grenzen frei wählbar. Vorteilhafterweise sind mindestens zwei Bauteile aus der Gruppe Oberteil, Messer, Schneidplatte und Unterteil mittels einer Klemmverbindung aneinander befestigbar. Hierdurch ist eine lösbare Verbindung zwischen mindestens zwei Bauteilen der Probennahme-Vorrichtung auf konstruktiv einfache Weise realisiert. Die Gestaltung der Klemmverbindung und insbesondere der Klemmen in Bezug auf Form, Material, Anordnung und Anzahl bestimmt dabei eine Montagebeziehungsweise Anpresskraft beim Schneidvorgang. Optional kann die Klemmverbindung für verschiedene Kulturpflanzen modifiziert ausgestaltet sein.

Eine vorteilhafte Weiterbildung der vorgenannten Ausführungsform der Erfindung sieht vor, dass das Unterteil und die Schneidplatte mittels mindestens einer ersten Klemme und das Oberteil, das Messer und das Unterteil mit der daran angeklemmten Schneidplatte mittels mindestens einer zweiten Klemme miteinander verbindbar sind. Auf diese Weise ist es möglich, die Probennahme-Vorrichtung während der bestimmungsgemäßen Verwendung lediglich teilweise zu demontieren oder im Zuge der Herstellung teilmontiert bereitzustellen. Beispielsweise ist dies sinnvoll, wenn das mit den gefüllten Probenbehältern bestückte Unterteil zur labortechnischen Untersuchung verbracht werden soll.

Eine weitere besonders vorteilhafte Weiterbildung der erfindungsgemäßen Lehre sieht vor, dass das Oberteil eine Basisplatte und einen die Anzuchtbehälter tragenden Aufsatz aufweist, wobei der Aufsatz und die Basisplatte in der Gebrauchslage der Probennahme-Vorrichtung miteinander lösbar verbunden sind. Hierdurch ist es beispielsweise möglich, als Zukaufteile ausgebildete Aufsätze, unter anderem als mit Nährstoffen vorkonfektionierte Zukaufteile, zu verwenden.

Eine vorteilhafte Weiterbildung der vorgenannten Ausführungsform sieht vor, dass die Bodenöffnung jedes Anzuchtbehälters des Aufsatzes außenseitig mit einem rohrartigen Kragen umgeben ist, wobei der Kragen in der Gebrauchslage der Probennahme-Vorrichtung im Wesentlichen bis an das dem Aufsatz abgewandte Ende der Basisplatte reicht. Auf diese Weise ist der Kontakt mit der Basisplatte des Oberteils wirksam verhindert.

Nach einer Weiterbildung der Erfindung kann zusätzlich ein Stanzstempel vorgesehen sein. Aus einer Basisplatte des Stanzstempels erheben sich Stifte, die jeweils einen Positionierkopf aufweisen können. Die Anzahl der Stifte stimmt mit der Anzahl der Probenbehälter und damit mit der Anzahl der Anzuchtbehälter überein. Der Stanzstempel ist vorzugsweise aus Metall hergestellt. Beispielsweise kann der Stanzstempel aus einem Kunststoff hergestellt sein. Der Stanzstempel dient dazu, eine Kreuzkontamination der Wurzelproben beim Abheben der Schneidplatte von dem Unterteil sicher zu vermeiden. Hierzu werden, nach dem Schneiden der Wurzeln und dem Entfernen des einteiligen oder mehrteiligen Oberteils, mittels des Stanzstempels rund um die einzelnen Schneidlöcher ringförmige, insbesondere kreisringförmige, Abschnitte aus der Schneidplatte herausgestanzt und in das Innere der jeweils zugeordneten Probenbehälter überführt. Hierfür wird der Stanzstempel mit der Schneidplatte in Eingriff gebracht. Um eine sichere Ausrichtung des Stanzstempels mit dessen Stiften zu der Schneidplatte und den Schneidlöchern zu gewährleisten und zu erleichtern, können an den einzelnen Stiften Positionierköpfe vorgesehen sein, die bei der Annäherung des Stanzstempels an die Schneidplatte zuerst in Eingriff mit den Schneidlöchern der Schneidplatte gelangen. Bei der weiteren Bewegung des Stanzstempels in Richtung Schneidplatte gelangen die Stifte des Stanzstempels in Anlage mit Rändern der Schneidlöcher der Schneidplatte. Die Dicke der Schneidplatte kann an den Rändern der Schneidlöcher geschwächt sein, so dass der Stanzstempel bei der weiteren Bewegung in Richtung der Schneidplatte die so gebildeten Sollbruchstellen der Schneidplatte bricht. Die einzelnen Stifte des Stanzstempels weisen eine entsprechende Dimensionierung auf, um die Ränder der Schneidlöcher mit den darin ausgebildeten Schneidlöchern bei der beschriebenen Bewegung des Stanzstempels in Richtung der Schneidplatte sicher in das Innere des jeweils korrespondierenden Probenbehälters zu überführen. Dies ermöglicht ein anschließendes Abheben der Schneidplatte von dem Unterteil, ohne dass Wurzelteile aus den einzelnen Probenbehältern des Unterteils in ungewünschter Weise herausgezogen werden.

Eine andere Weiterbildung kann eine modifizierte Schneidplatte vorsehen. An der dem Unterteil zugewandten Unterseite der modifizierten Schneidplatte können als Nuten ausgebildete Entwässerungsöffnungen ausgebildet sein. Jede der Entwässerungsöffnungen ist dabei genau einem Probenbehälter zugeordnet, um so eine unerwünschte Kreuzkontamination wirksam zu verhindern.

In zur erfindungsgemäßen Probennahme-Vorrichtung gehörenden Anzuchtbehältern werden Pflanzen gezogen. Hierfür sind die Anzuchtbehälter mit Nährstoffen für das Pflanzenwachstum befüllt. Um die erforderliche Menge an Wasser für das Wachstum zu speichern, kann in den Anzuchtbehältern ein Granulat oder dergleichen eingebracht sein. Die Anzuchtbehälter werden gewässert, um so das Granulat mit Wasser zu tränken, welches dann von dem Granulat an die Pflanzen abgegeben wird. Bei dem Wässern kann es vorkommen, dass ein Überschuss an Wasser den einzelnen Anzuchtbehältern zugeführt wird. Dieses Wasser kann nicht von dem Granulat aufgenommen werden; es läuft über die Bodenöffnungen der betroffenen Anzuchtbehälter in die diesen zugeordneten Probenbehälter. Für die weitere genetische Untersuchung ist es unschädlich, dass sich in den Probenbehältern Wasser befindet. Jedoch ist es ungewünscht, dass Anzuchtbehälter von Wasser geflutet sind. Deshalb sieht diese Weiterbildung eine modifizierte Schneidplatte mit Entwässerungsöffnungen, beispielsweise Nuten, vor. Die Entwässerungsöffnungen sind dabei derart an der Unterseite der Schneidplatte angeordnet, dass Wasser, welches von dem einzelnen Probenbehälter nicht aufgenommen werden kann, nicht in ungewünschter Weise in den dazu korrespondierenden Anzuchtbehälter aufsteigt, sondern über die Probenbehälteröffnung in die jeweilige Entwässerungsöffnung gelangt und über die Unterseite der Schneidplatte abfließen kann, ohne in ungewünschter Weise in einen der anderen Probenbehälter einzufließen. Auf diese Weise werden Staunässe und etwaige unerwünschte Kontaminationen wirksam vermieden.

Aus den weiteren Unteransprüchen und der nachfolgenden Beschreibung sind weitere Vorteile, Merkmale und Einzelheiten der Erfindung zu entnehmen. Dort erwähnte Merkmale können jeweils einzeln für sich oder auch in beliebiger Kombination erfindungswesentlich sein. So kann auf die Offenbarung zu den einzelnen Erfindungsaspekten stets wechselseitig Bezug genommen werden. Die Zeichnungen dienen lediglich beispielhaft der Klarstellung der Erfindung und haben keinen einschränkenden Charakter. Es zeigen:
- Fig. 1: ein erstes Ausführungsbeispiel einer erfindungsgemäßen Probennahme-Vorrichtung in einer ersten perspektivischen Explosionsdarstellung;

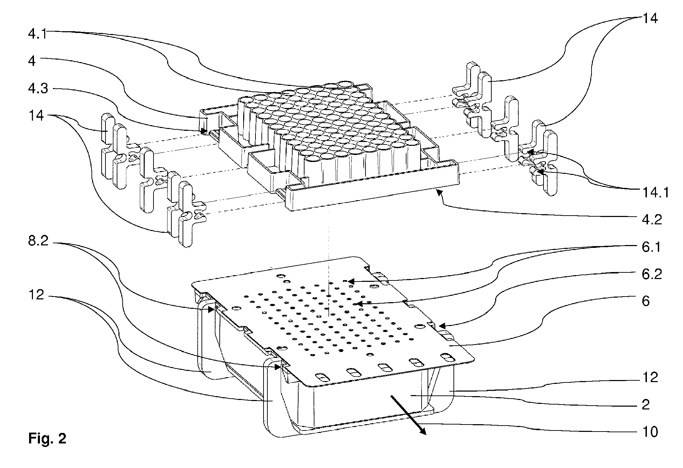

- Fig. 2: das erste Ausführungsbeispiel in einer zweiten perspektivischen Explosionsdarstellung;

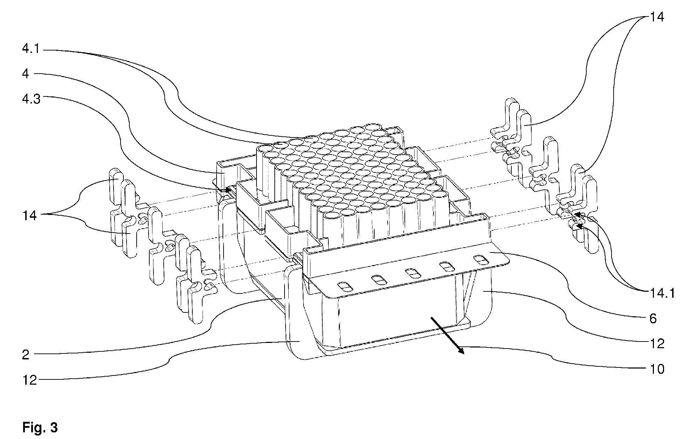

- Fig. 3: das erste Ausführungsbeispiel in einer dritten perspektivischen Explosionsdarstellung;
- Fig. 4: das erste Ausführungsbeispiel in einer ersten perspektivischen Zusammenbaudarstellung;
- Fig. 5: das erste Ausführungsbeispiel in einer zweiten perspektivischen Zusammenbaudarstellung;
- Fig. 6: das erste Ausführungsbeispiel der erfindungsgemäßen Probennahme-Vorrichtung in einer Draufsicht;
- Fig. 7: ein zweites Ausführungsbeispiel in einer zur Fig. 4 korrespondierenden perspektivischen Zusammenbaudarstellung;
- Fig. 8: ein drittes Ausführungsbeispiel in einer ersten perspektivischen Explosionsdarstellung in teilweiser Ansicht;
- Fig. 9: das dritte Ausführungsbeispiel in einer zweiten perspektivischen Explosionsdarstellung in teilweiser Ansicht;
- Fig. 10: das dritte Ausführungsbeispiel in einer Detailansicht im Bereich der Schneidplatte in einer geschnittenen, teilweisen Ansicht;
- Fig. 11: das dritte Ausführungsbeispiel in einer ersten perspektivischen Eingriffsdarstellung in teilweiser Ansicht und
- Fig. 12: ein viertes Ausführungsbeispiel in einer ersten perspektivischen Explosionsdarstellung in teilweiser Ansicht.

Die Figuren zeigen voneinander verschiedene Ausführungsbeispiele der erfindungsgemäßen Probennahme-Vorrichtung. Gleiche oder gleichwirkende Bauteile sind mit gleichen Bezugszeichen bezeichnet. Es werden lediglich die Unterscheidungsmerkmale der auf das erste Ausführungsbeispiel folgenden Ausführungsbeispiele zu dem ersten Ausführungsbeispiel erläutert. Im Übrigen stimmen die Ausführungsbeispiele überein.

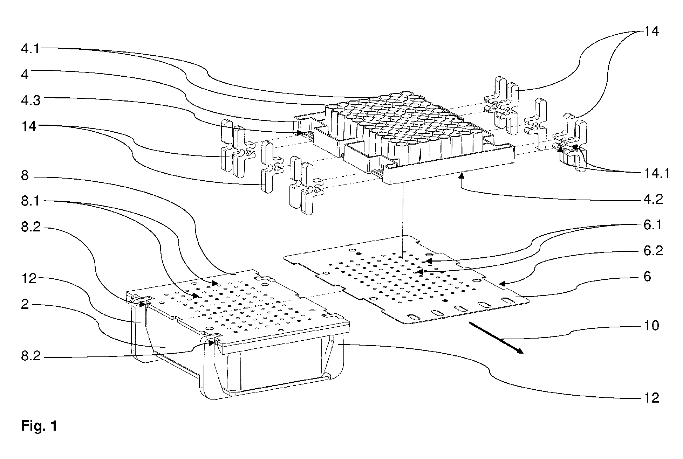

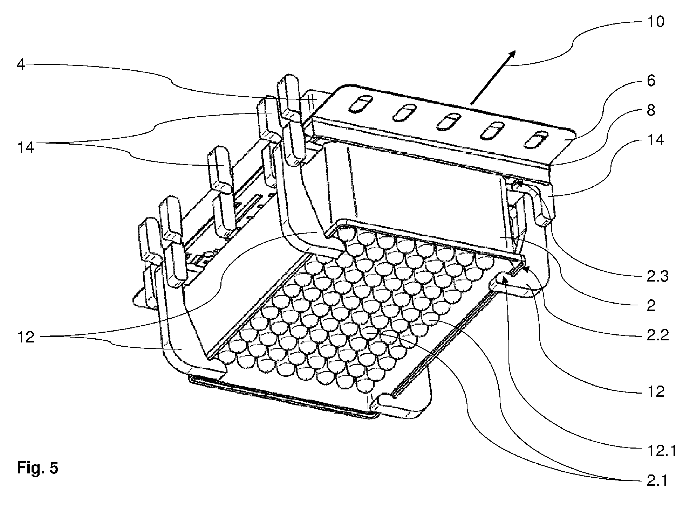

Fig. 1 zeigt ein erstes Ausführungsbeispiel einer erfindungsgemäßen Probennahme-Vorrichtung in einer perspektivischen Explosionsdarstellung. Die Probennahme-Vorrichtung weist ein bevorzugt jedenfalls in Teilen aus Kunststoff gebildetes Unterteil 2 mit einer Mehrzahl von Probenbehältern 2.1 auf, die besonders deutlich in einer perspektivischen Unterseitenansicht der Probennahme-Vorrichtung nach Fig. 5 erkennbar sind.

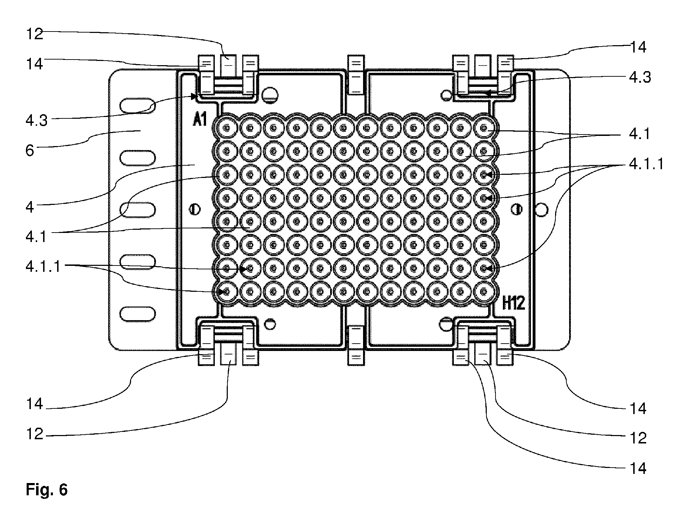

Ferner weist die Probennahme-Vorrichtung ein aus Kunststoff hergestelltes Oberteil 4 mit einer Mehrzahl von Anzuchtbehältern 4.1, ein bevorzugt aus Kunststoff oder Metall hergestelltes Messer 6 und eine bevorzugt aus Kunststoff hergestellte Schneidplatte 8 auf, wobei in jedem Anzuchtbehälter 4.1 jeweils eine Bodenöffnung 4.1.1 ausgebildet ist, die in Fig. 6 zu erkennen sind. Die Probenbehälter 2.1 sind hier integraler Bestandteil des Unterteils 2 und die Anzuchtbehälter 4.1 sind integraler Bestandteil des Oberteils 4. Das Unterteil 2 und das Oberteil 4 sind in dem ersten Ausführungsbeispiel der Erfindung also jeweils einstückig ausgebildet.

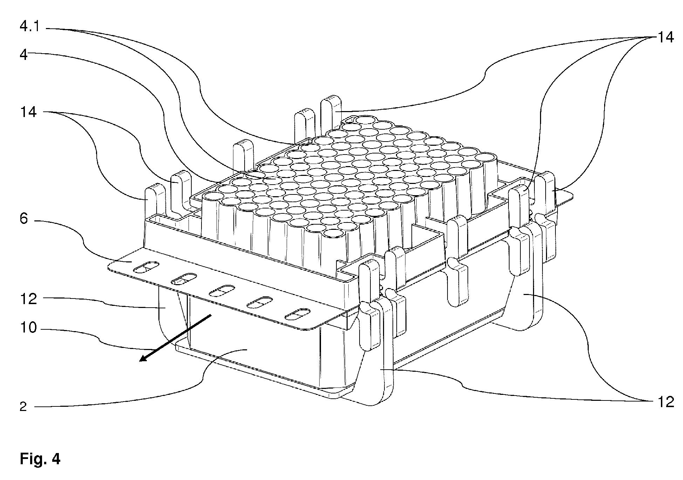

Wie aus der Zusammenschau der Figuren deutlich wird, ist jedem Anzuchtbehälter 4.1 genau ein Probenbehälter 2.1 und jeder Bodenöffnung 4.1.1 genau eine Probenbehälteröffnung in einer in den Fig. 4 bis 6 dargestellten Gebrauchslage zugeordnet. Die Probenbehälteröffnungen sind nicht explizit dargestellt, da sie von der Schneidplatte 8 überdeckt sind.

Die eindeutige Zuordnung von genau einem Anzuchtbehälter 4.1 zu genau einem Probenbehälter 2.1 ist hier deshalb sinnvoll, weil die Probennahme-Vorrichtung hier insbesondere für genetische Untersuchungen von Pflanzen vorgesehen ist. Für den Erfolg derartiger Untersuchungen ist es wichtig, dass es zu keiner ungewünschten Durchmischung (Kreuzkontamination) der einzelnen Gewebeproben (Genotypen) kommt. Dies ist durch die vorgenannte Ausbildung der Probennahme-Vorrichtung gewährleistet.

Die Anzuchtbehälter 4.1 wurden hier vorab mit nicht dargestellten Nährstoffen für das Pflanzenwachstum befüllt. In den einzelnen Anzuchtbehältern 4.1 wird im Gebrauch der erfindungsgemäßen Probennahme-Vorrichtung je eine Pflanze gezogen. Die Wurzeln der nicht dargestellten Pflanzen wachsen im weiteren Verlauf der Pflanzenentwicklung durch die Bodenöffnungen 4.1.1 der Anzuchtbehälter 4.1 und die Probenbehälteröffnungen in die zu den einzelnen Anzuchtbehältern 4.1 korrespondierenden Probenbehälter 2.1 des Unterteils 2 hinein.

Das Messer 6 und die Schneidplatte 8 sind in der in den Fig. 4 bis 6 dargestellten Gebrauchslage der erfindungsgemäßen Probennahme-Vorrichtung derart zwischen dem Oberteil 4 und dem Unterteil 2 angeordnet, dass die durch die Bodenöffnungen 4.1.1 der Anzuchtbehälter 4.1 heraus- und durch die Probenbehälteröffnungen in den jeweiligen Probenbehälter 2.1 hineinragenden Wurzeln mittels des Messers 6 und der Schneidplatte 8 durchtrennbar sind.

Das Messer 6 und die Schneidplatte 8 sind hier als eine Messerlochplatte 6 und eine Loch-Schneidplatte 8 ausgebildet. Dabei ist die Anzahl von in den beiden Lochplatten 6, 8 ausgebildeten Schneidlöchern 6.1, 8.1 identisch mit der Anzahl der Bodenöffnungen 4.1.1 der Anzuchtbehälter 4.1 und der Anzahl der Probenbehälteröffnungen der Probenbehälter 2.1. Die Aufzuchtbehälter 4.1 und die Probenbehälter 2.1 sind bevorzugt aus Kunststoff gebildet.

In der in den Fig. 4 bis 6 dargestellten Gebrauchslage sind die an dem Messer 6 und der Schneidplatte 8 ausgebildeten Schneidlöcher 6.1, 8.1 deckungsgleich mit den dazu korrespondierenden Bodenöffnungen 4.1.1 und Probebehälteröffnungen. Entsprechend können die Wurzeln der in den Anzuchtbehältern 4.1 gezogenen Pflanzen ungehindert von dem jeweiligen Anzuchtbehälter 4.1 durch die Bodenöffnungen 4.1.1, die Schneidlöcher 6.1, 8.1 und die Probenbehälteröffnungen in den jeweiligen Probenbehälter 2.1 hineinwachsen.

Zur Durchtrennung der nicht dargestellten Wurzeln wird das Messer 6 entlang der Schneidplatte 8 in Richtung des Pfeiles 10 geführt, so dass die Wurzeln zwischen dem Messer 6 und der Schneidplatte 8, also an den Kanten der Schneidlöcher 6.1, 8.1, abgeschert werden. Um die Bewegung des Messers 6 parallel zum Pfeil 10 zu ermöglichen, weist das Messer 6 an dessen beiden Längsseiten Längsausnehmungen 6.2 auf, was nachfolgend noch näher erläutert ist.

Die Schneidplatte 8 ist vorliegend als ein abnehmbarer Deckel 8 für das Unterteil 2 ausgebildet, wobei die Schneidplatte 8 mittels erster Klemmen 12 an dem Unterteil 2 lösbar befestigt ist. Wie insbesondere aus den Fig. 1 und 5 ersichtlich ist, umfassen die ersten Klemmen 12 das Unterteil 2 und die Schneidplatte 8 klammerartig und rasten mit an den freien Enden der ersten Klemmen 12 ausgebildeten Rastvorsprüngen 12.1 in dazu korrespondierend ausgebildete Rastaufnahmen 8.2 der Schneidplatte 8 und hinter einen an dem Unterteil 2 ausgebildeten Rastkragen 2.2 ein.

Damit die ersten Klemmen 12 die Bewegung des Messers 6 entlang der Schneidplatte 8 nicht behindern, überragt die Schneidplatte 8 die ersten Klemmen 12 in der in Fig. 1 dargestellten Gebrauchslage von dem Unterteil 2 und der Schneidplatte 8.

Ferner ist die Baugruppe, die aus dem Unterteil 2 und der mittels der ersten Klemmen 12 daran befestigten Schneidplatte 8 gebildet ist, mit dem Messer 6 und dem Oberteil 4 durch zweite Klemmen 14 lösbar verbunden. Hierfür sind die vorgenannten Bauteile passgenau übereinandergelegt und mit den zweiten Klemmen 14 geklemmt. Analog zu der Klemmverbindung zwischen dem Unterteil 2 und der Schneidplatte 8 weisen das Unterteil 2 und das Oberteil 4 Rastaufnahmen 2.3 und 4.3 auf, die mit an freien Enden der zweiten Klemmen 14 ausgebildeten Rastvorsprüngen 14.1 in der in den Fig. 4 bis 6 dargestellten Gebrauchslage eine lösbare Rastverbindung eingehen.

Zur besseren Führung des Messers 6 zwischen der an dem Unterteil 2 angeordneten Schneidplatte 8 und dem Oberteil 4 ist der dem Messer 6 zugewandte Boden 4.2 des Oberteils 4 als eine Messerführung 4.2 abschnittsweise eben beziehungsweise plan ausgebildet.

Die erfindungsgemäße Probennahme-Vorrichtung gemäß dem ersten Ausführungsbeispiel ist derart ausgebildet, dass sich das Messer 6 relativ zu der Schneidplatte 8 und parallel zu dem Pfeil 10 bewegen lässt, trotz der durch die ersten und zweiten Klemmen 12, 14 zwischen dem Oberteil 4, dem Messer 6, der Schneidplatte 8 und dem Unterteil 2 gebildeten Klemmverbindungen.

Dies ist unter anderem deshalb möglich, weil an den beiden Längsseiten des Messers 6 Längsausnehmungen 6.2 ausgebildet sind. Entsprechend wird eine Bewegung des Messers 6 parallel zu dem Pfeil 10 und in dem erforderlichen Bewegungsbereich durch die zweiten Klemmen 14 nicht behindert. Eine in Richtung des Pfeils 10 (Betätigungsrichtung des Messers 6) bestimmter Messerhub ist dabei durch die Länge der Längsausnehmungen 6.2 bestimmt. Die Zuordnung der Schneidlöcher 6.1 des Messers 6 einerseits zu den Schneidlöchern 8.1 der Schneidplatte 8, den Bodenöffnungen 4.1.1 und den Probenbehälteröffnungen andererseits ist dabei bevorzugt so gewählt, dass in einer ersten Hubendstellung des Messers 6 die Schneidlöcher 6.1, 8.1 derart übereinander liegen, sodass die Pflanzen während der Pflanzenentwicklung ungehindert von dem Aufzuchtbehälter 4.1 in den Probenbehälter 2.1 wachsen können, und dass die Wurzeln der Pflanzen in der zweiten Hubendstellung durchtrennt sind. Das Durchtrennen der Wurzeln erfolgt hierbei kontaminationsfrei, sofern der Messerhub kleiner gewählt ist als ein in die Betätigungsrichtung 10 des Messers 6 bestimmter Abstand benachbarter Schneidlöcher 6.1, 8.1.

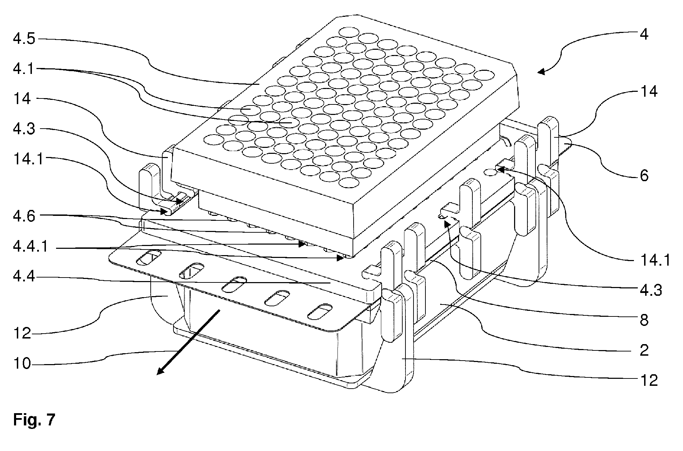

In Fig. 7 ist ein zweites Ausführungsbeispiel der erfindungsgemäßen Probennahme-Vorrichtung in einer Zusammenbaulage gezeigt. Im Unterschied zu dem ersten Ausführungsbeispiel weist das zweite Ausführungsbeispiel ein zweiteiliges Oberteil 4 auf. Das Oberteil 4 umfasst hier eine Basisplatte 4.4 und einen die Anzuchtbehälter 4.1 tragenden Aufsatz 4.5, wobei der Aufsatz 4.5 und die Basisplatte 4.4 in einer in Fig. 7 dargestellten Gebrauchslage der Probennahme-Vorrichtung miteinander lösbar verbunden sind.

Die Zweiteilung des Oberteils 4 dient dazu, eine Mehrfachbenutzung der Probennahme-Vorrichtung zu erleichtern oder den Aufsatz 4.5 mit den Anzuchtbehältern 4.1 als Zukaufteil zu verwenden.

Bei dem vorliegenden Ausführungsbeispiel ist die Bodenöffnung jedes Anzuchtbehälters 4.1 des Aufsatzes 4.5 außenseitig mit einem rohrartigen Kragen 4.6 umgeben, wobei der Kragen 4.6 in der in Fig. 7 dargestellten Gebrauchslage der Probennahme-Vorrichtung im Wesentlichen bis an das dem Aufsatz 4.5 abgewandte Ende der Basisplatte 4.4 reicht.

Um den Aufsatz 4.5 an der Basisplatte 4.4 zu befestigen, wird der Aufsatz 4.5 mit den rohrartigen Kragen 4.6 in an der Basisplatte 4.4 ausgebildete Durchgangslöcher 4.4.1 gesteckt. Die Kragen 4.6 und die dazu korrespondierenden Durchgangslöcher 4.4.1 sorgen gleichzeitig für eine Positionierung des Aufsatzes 4.5 zu der Bodenplatte 4.4.

Der Aufsatz 4.5 und die Basisplatte 4.4 sind bevorzugt aus Kunststoff hergestellt.

Die auf diese Weise gebildete Baueinheit aus Bodenplatte 4.4 und dem die Anzuchtbehälter 4.1 aufweisenden Aufsatz 4.5 ist in der in Fig. 7 dargestellten Gebrauchslage, wie im Rahmen des ersten Ausführungsbeispiels bereits erläutert, mittels zweiter Klemmen 14 mit den übrigen Bauteilen, nämlich dem die Probenbehälter 2.1 aufweisenden Unterteil 2, dem Messer 6 und der Schneidplatte 8 lösbar verbunden. Analog zu dem ersten Ausführungsbeispiel sind das Unterteil 2 und die Schneidplatte 8 auch hier vorab mittels erster Klemmen 12 miteinander verbunden.

Zwecks Durchtrennung der in die Probenbehälter 2.1 hineinragenden Wurzeln kann das Messer 6 bei beiden Ausführungsbeispielen sowohl manuell wie auch mittels eines Motorantriebs längs der Schneidplatte 8 bewegt werden. Der Fachmann wird je nach Anwendungsfall die geeigneten Maßnahmen ergreifen.

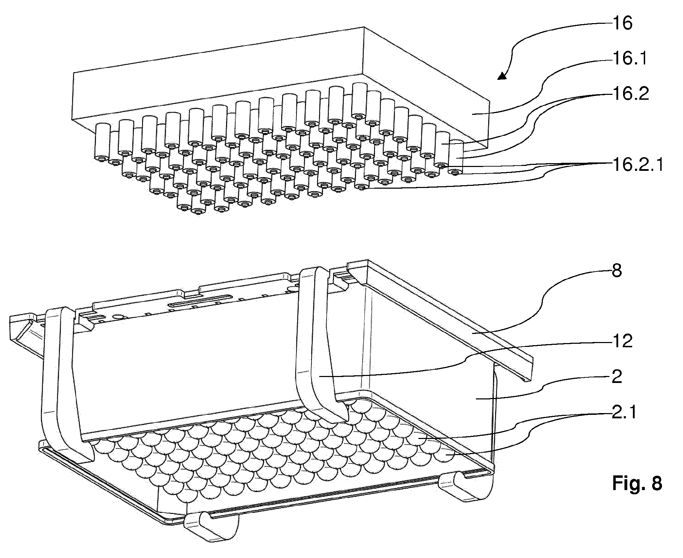

Anhand der Fig. 8 bis 11 ist ein drittes Ausführungsbeispiel der erfindungsgemäßen Lehre erläutert.

Fig. 8 zeigt das dritte Ausführungsbeispiel in einer perspektivischen Explosionsdarstellung in teilweiser Ansicht. Dargestellt ist die Baugruppe aus dem Unterteil 2 mit den Probenbehältern 2.1, den ersten Klemmen 12 und der Schneidplatte 8. Das Unterteil 2 mit den Probenbehältern 2.1 und die ersten Klemmen 12 wie auch die nicht dargestellten Bauteile der Probennahme-Vorrichtung, wie beispielsweise das Oberteil 4 und das Messer 6, können analog zu dem ersten oder dem zweiten Ausführungsbeispiel ausgebildet sein.

Im Unterschied zu dem ersten und dem zweiten Ausführungsbeispiel weist die Probennahme-Vorrichtung des dritten Ausführungsbeispiels zusätzlich ein weiteres Bauteil auf, nämlich einen Stanzstempel 16. Aus einer Basisplatte 16.1 des Stanzstempels 16 erheben sich Stifte 16.2, die jeweils einen Positionierkopf 16.2.1 aufweisen. Die Anzahl der Stifte 16.2 stimmt mit der Anzahl der Probenbehälter 2.1 und damit mit der Anzahl der hier nicht dargestellten Anzuchtbehälter überein. Der Stanzstempel 16 ist vorzugsweise aus einem metallischen Werkstoff oder aus Kunststoff hergestellt.

Der Stanzstempel 16 dient dazu, eine Kreuzkontamination der Wurzelproben beim Abheben der Schneidplatte 8 sicher zu vermeiden. Hierzu werden, nach dem Schneiden der Wurzeln und dem Entfernen des nicht dargestellten einteiligen oder zweiteiligen Oberteils, mittels des Stanzstempels 16 rund um die einzelnen Schneidlöcher, hier ebenfalls nicht dargestellt, kreisringförmige Abschnitte aus der Schneidplatte 8 herausgestanzt und in das Innere der jeweils zugeordneten Probenbehälter 2.1 überführt.

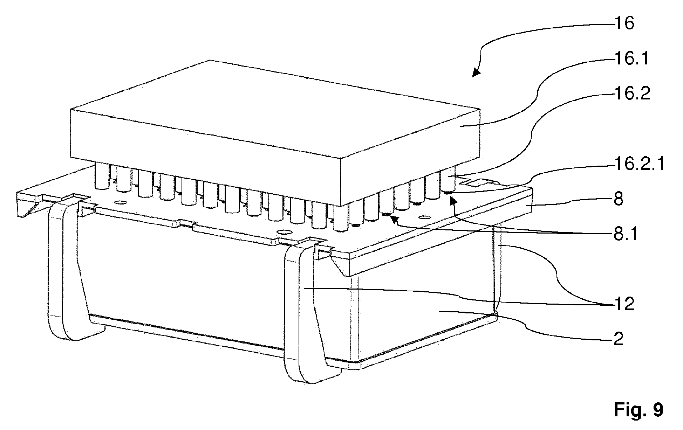

Wie in Fig. 9 dargestellt wird hierfür der Stanzstempel 16 mit der Schneidplatte 8 in Eingriff gebracht. Um eine sichere Ausrichtung des Stanzstempels 16 mit dessen Stiften 16.2 zu der Schneidplatte 8 und den Schneidlöchern 8.1 zu gewährleisten und zu erleichtern, gelangen bei der Annäherung des Stanzstempels 16 an die Schneidplatte 8 zunächst die an den Stiften 16.2 ausgebildeten Positionierköpfe 16.2.1 in Eingriff mit den Schneidlöchern 8.1 der Schneidplatte 8. Bei der weiteren Bewegung des Stanzstempels 16 in Richtung Schneidplatte 8 gelangen die Stifte 16.2 des Stanzstempels 16 in Anlage mit Rändern der Schneidlöcher 8.1 der Schneidplatte 8.

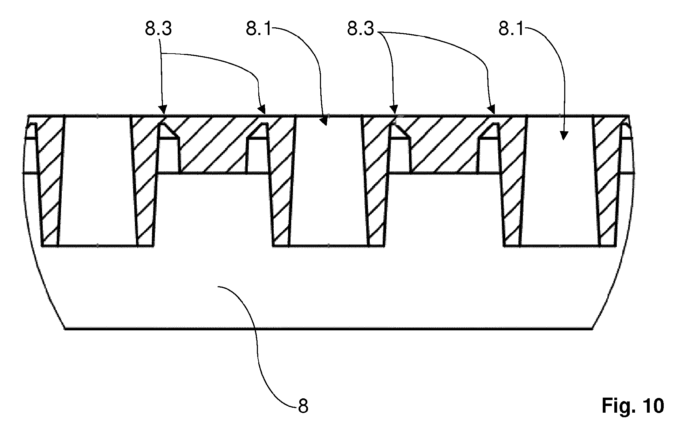

Siehe hierzu Fig. 10, in der ein Detail der Probennahme-Vorrichtung gemäß des dritten Ausführungsbeispiels im Bereich der Schneidplatte 8 gezeigt ist. Deutlich zu erkennen sind die vorgenannten Ränder 8.3, die die Schneidlöcher 8.1 der Schneidplatte 8 umgeben. Die Dicke der Schneidplatte 8 ist an den Rändern 8.3 geschwächt, so dass der hier nicht dargestellte Stanzstempel bei der weiteren Bewegung in Richtung der Schneidplatte 8 die so gebildeten Sollbruchstellen der Schneidplatte 8 bricht. Der Stanzstempel wird bei der Bewegung in Richtung der Schneidplatte 8 in der Bildebene von Fig. 10 manuell oder motorisch von oben auf die Schneidplatte 8 abgesenkt.

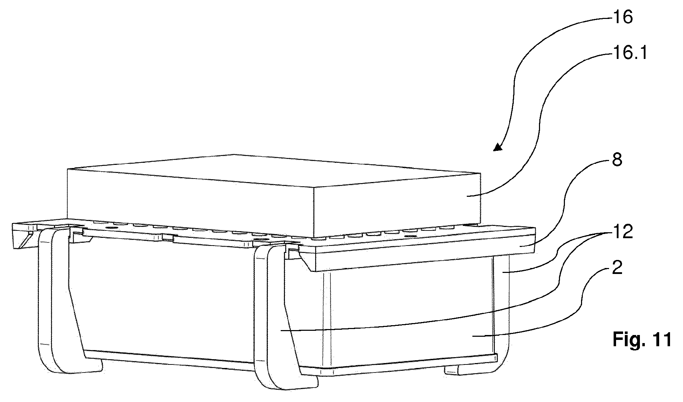

Die einzelnen Stifte des Stanzstempels weisen eine entsprechende Dimensionierung auf, um die Ränder 8.3 der Schneidplatte 8 mit den darin ausgebildeten Schneidlöchern 8.1 bei der beschriebenen Bewegung des Stanzstempels in Richtung der Schneidplatte 8 sicher in das Innere des jeweils korrespondierenden Probenbehälters zu überführen und in dem Probenbehälter zu halten, um ein ungewünschtes Herausziehen von Wurzelproben aus den Probenbehältern bei einer Entfernung der Schneidplatte 8 von dem Unterteil und damit von den Probenbehältern wirksam zu verhindern. Das Unterteil und die Probenbehälter sind in Fig. 10 ebenfalls nicht dargestellt. Fig. 11 zeigt den Stanzstempel 16 in der Endlage, in der der Stanzstempel 16 mit dessen Basisplatte 16.1 an der Schneidplatte 8 anliegt. Der Übersichtlichkeit wegen ist die Basisplatte 16.1 in der Fig. 11 nicht vollständig auf die Schneidplatte 8 abgesenkt dargestellt.

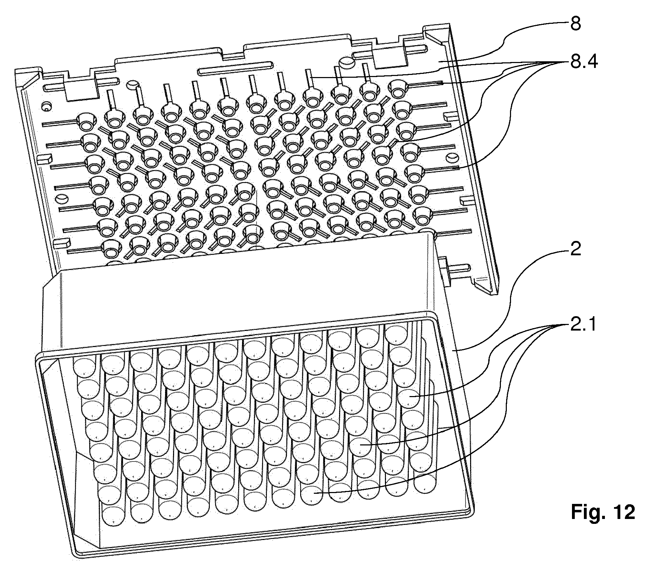

In Fig. 12 ist ferner ein viertes Ausführungsbeispiel dargestellt. Gezeigt ist das Unterteil 2 mit den Probenbehältern 2.1 und die Schneidplatte 8 in einer perspektivischen Unteransicht. Das Unterteil 2 mit den Probenbehältern 2.1 wie auch die nicht dargestellten Bauteile der Probennahme-Vorrichtung, wie beispielsweise das Oberteil 4 und das Messer 6, können analog zu dem ersten oder dem zweiten oder dem dritten Ausführungsbeispiel ausgebildet sein.

Im Unterschied zu den bereits erläuterten Ausführungsbeispielen weist die Probennahme-Vorrichtung des vierten Ausführungsbeispiels eine modifizierte Schneidplatte 8 auf. Wie aus Fig. 12 ersichtlich ist, sind in der Unterseite der Schneidplatte 8 als Nuten ausgebildete Entwässerungsöffnungen 8.4 ausgebildet. Jede der Entwässerungsöffnungen 8.4 ist dabei in der nicht dargestellten Zusammenbaulage von Unterteil 2 und Schneidplatte 8 genau einem Probenbehälter 2.1 zugeordnet, um so eine ungewünschte Kreuzkontamination wirksam zu verhindern.

Wie bereits anhand des ersten Ausführungsbeispiels erläutert, werden in den in Fig. 12 nicht dargestellten Anzuchtbehältern Pflanzen gezogen. Hierfür sind die Anzuchtbehälter mit Nährstoffen für das Pflanzenwachstum befüllt. Um die erforderliche Menge an Wasser für das Wachstum zu speichern, kann in den Anzuchtbehältern ein Granulat oder dergleichen eingebracht sein. Die Anzuchtbehälter werden gewässert, um so das Granulat mit Wasser zu tränken, welches dann von dem Granulat an die Pflanzen abgegeben wird. Bei dem Wässern kann es vorkommen, dass ein Überschuss an Wasser den einzelnen Anzuchtbehältern zugeführt wird. Dieses Wasser kann nicht von dem Granulat aufgenommen werden; es läuft über die Bodenöffnungen der betroffenen Anzuchtbehälter in die diesen zugeordneten Probenbehälter 2.1.

Für die weitere insbesondere genetische Untersuchung ist es unschädlich, dass sich in den Probenbehältern 2.1 Wasser befindet. Jedoch ist es ungewünscht, dass Anzuchtbehälter von Wasser geflutet sind. Deshalb ist in dem vierten Ausführungsbeispiel für jeden Probenbehälter 2.1 eine als Nut ausgebildete Entwässerungsöffnung 8.4 vorgesehen. Die Nuten 8.4 sind dabei derart an der Unterseite der Schneidplatte 8 angeordnet, dass Wasser, welches von dem einzelnen Probenbehälter 2.1 nicht aufgenommen werden kann, nicht in ungewünschter Weise in den dazu korrespondierenden Anzuchtbehälter aufsteigt, sondern über die nicht dargestellte Probenbehälteröffnung in die jeweilige Nut 8.4 gelangt und über die Unterseite der Schneidplatte 8 abfließen kann, ohne in ungewünschter Weise in einen der anderen Probenbehälter 2.1 einzufließen. Auf diese Weise ist Staunässe wirksam vermieden.

Die Erfindung ist nicht auf die vorgenannten Ausführungsbeispiele begrenzt.

Um die einzelnen Bauteile der Probennahme-Vorrichtung, beispielsweise Unterteil, Oberteil, Messer und Schneidplatte, mit weniger Kontrollaufwand zueinander sicher ausrichten zu können, können die Bauteile auch bei nicht zweigeteiltem Oberteil zumindest teilweise zueinander korrespondierende Positioniermittel aufweisen.

Die Positioniermittel können ferner als Codierung ausgebildet sein, durch die eine fehlerhafte Montage der Bauteile der erfindungsgemäßen Probennahme-Vorrichtung mit einfachen Mitteln wirksam verhindert ist.

Bei den genannten Ausführungsbeispielen sind die Probenbehälter des Unterteils und die Anzuchtbehälter des Oberteils jeweils integraler Bestandteil des Unter- oder Oberteils. Dies ist jedoch nicht zwingend erforderlich. Beispielsweise kann es auch vorgesehen sein, dass die Probenbehälter und/oder die Anzuchtbehälter zumindest teilweise als separates Bauteil ausgebildet sind.

Die erfindungsgemäße Probennahme-Vorrichtung kann sowohl für den einmaligen Gebrauch wie auch für den mehrmaligen Gebrauch ausgelegt sein. Während die erste Ausführungsform eher als Einweg-Probenahme-Vorrichtung geeignet ausgebildet ist, eignet sich die zweite Ausführungsform besser für die mehrfache Verwendung.

Im Unterschied zu den Ausführungsbeispielen wäre es grundsätzlich denkbar, dass ein Anzuchtbehälter mit dessen Bodenöffnung nicht zwingend genau zu einem Probenbehälter und dessen Probenbehälteröffnung korrespondiert. Möglich wäre auch, dass ein Anzuchtbehälter mit dessen Bodenöffnung einer Mehrzahl von Probenbehältern und deren Probenbehälteröffnungen zugeordnet ist. Gleiches Pflanzenmaterial kann so unterschiedlichen Untersuchungen zugeführt werden.

Das Messer muss nicht zwingend als eine Lochplatte ausgebildet sein. Denkbar ist auch, dass beispielsweise lediglich die Schneidplatte als eine Lochplatte ausgebildet ist und das Messer von dem Fachmann je nach dem vorliegenden Einzelfall nach Art, Material, Form, Dimensionierung und Anordnung anders geeignet ausgewählt ist.

Beispielsweise wären, neben anderen geeigneten Materialien, Messer aus gehärteten Werkzeugstahl, aus legiertem Werkzeugstahl, aus Hartmetall, aus Kunststoff oder auch aus Schneidkeramik möglich. Gleiches gilt für das Material der Schneidplatte.

In den beiden Ausführungsbeispielen wirken die zweiten Klemmen 14 in der Gebrauchslage der erfindungsgemäßen Probennahme-Vorrichtung unter anderem mit an dem Unterteil 2 ausgebildeten Rastaufnahmen 2.3 zusammen. Da das Unterteil 2 und die Schneidplatte 8 jedoch mit ersten Klemmen 12 miteinander lösbar verbunden sind, wäre es auch denkbar, dass die zweiten Klemmen mit an der Schneidplatte ausgebildeten Rastaufnahmen zusammenwirken.

### Bezugszeichenliste

- 2: Unterteil
- 2.1: Probenbehälter
- 2.2: Rastkragen des Unterteils
- 2.3: Rastaufnahmen des Unterteils
- 4: Oberteil
- 4.1: Anzuchtbehälter
- 4.1.1: Bodenöffnung des Anzuchtbehälters
- 4.2: Boden des Oberteils, als Messerführung für das Messer ausgebildet
- 4.3: Rastaufnahmen des Oberteils
- 4.4: Basisplatte des Oberteils
- 4.4.1: Durchgangslöcher der Basisplatte
- 4.5: Aufsatz des Oberteils
- 4.6: Kragen, der die Bodenöffnung des Anzuchtbehälters umgibt
- 6: Messer, als Messerlochplatte ausgebildet
- 6.1: Schneidlöcher des Messers
- 6.2: Längsausnehmungen des Messers
- 8: Schneidplatte, als Loch-Schneidplatte ausgebildet
- 8.1: Schneidlöcher der Schneidplatte
- 8.2: Rastaufnahmen der Schneidplatte
- 8.3: Ränder der Schneidplatte, die die Schneidlöcher umgeben
- 8.4: Entwässerungsöffnungen der Schneidplatte
- 10: Pfeil, der die Bewegungsrichtung des Messer bei der Durchtrennung der Wurzeln symbolisiert
- 12: Erste Klemmen
- 12.1: Rastvorsprünge der ersten Klemmen
- 14: Zweite Klemmen
- 14.1: Rastvorsprünge der zweiten Klemmen
- 16: Stanzstempel
- 16.1: Basisplatte des Stanzstempels
- 16.2: Stifte des Stanzstempels
- 16.2.1: Positionierköpfe der Stifte

## Patentansprüche

1. Probennahme-Vorrichtung für Pflanzen, **dadurch gekennzeichnet, dass** die Probennahme-Vorrichtung ein Unterteil (2) mit einer Mehrzahl von Probenbehältern (2.1), ein Oberteil (4) mit einer Mehrzahl von Anzuchtbehältern (4.1), ein Messer (6) und eine Schneidplatte (8) aufweist, wobei in jedem Anzuchtbehälter (4.1) eine Bodenöffnung (4.1.1) ausgebildet ist, die zu einer Probenbehälteröffnung eines Probenbehälters (2.1) korrespondiert, und das Messer (6) und die Schneidplatte (8) in einer Gebrauchslage der Probennahme-Vorrichtung derart zwischen dem Oberteil (4) und dem Unterteil (2) angeordnet sind, dass ein durch die Bodenöffnung (4.1.1) des Anzuchtbehälters (4.1) heraus- und durch die Probenbehälteröffnung in den Probenbehälter (2.1) hineinragendes Pflanzenteil mittels des Messers (6) und der Schneidplatte (8) durchtrennbar ist.

2. Probennahme-Vorrichtung nach Anspruch 1, **dadurch gekennzeichnet, dass** das Messer (6) und/oder die Schneidplatte (8) als eine Lochplatte (6, 8) ausgebildet ist/sind.

3. Probennahme-Vorrichtung nach Anspruch 1 oder 2, **dadurch gekennzeichnet, dass** die Schneidplatte (8) als ein Deckel (8) für das Unterteil (2) ausgebildet ist.

4. Probennahme-Vorrichtung nach einem der Ansprüche 1 bis 3, **dadurch gekennzeichnet, dass** eine dem Messer (6) zugewandte Fläche des Unterteils und/oder eine dem Messer zugewandte Fläche (4.2) des Oberteils (4) als eine Messerführung (4.2) ausgebildet ist/sind.

5. Probennahme-Vorrichtung nach einem der Ansprüche 1 bis 4, **dadurch gekennzeichnet, dass** mindestens zwei Bauteile aus der Gruppe Oberteil (4), Messer (6), Schneidplatte (8) und Unterteil (2) mittels einer Klemmverbindung (12, 14) aneinander befestigbar sind.

6. Probennahme-Vorrichtung nach Anspruch 5, **dadurch gekennzeichnet, dass** das Unterteil (2) und die Schneidplatte (8) mittels mindestens einer ersten Klemme (12) und das Oberteil (4), das Messer (6) und das Unterteil (2) mit der daran angeklemmten Schneidplatte (8) mittels mindestens einer zweiten Klemme (14) miteinander verbindbar sind.

7. Probennahme-Vorrichtung nach einem der Ansprüche 1 bis 6, **dadurch gekennzeichnet, dass** mindestens ein Bauteil aus der Gruppe Oberteil (4), Unterteil (2) und Schneidplatte (8) zu der ersten Klemme (12) und/oder der zweiten Klemme (14) korrespondierende Rastmittel (2.2, 2.3, 4.3, 8.2) aufweist.

8. Probennahme-Vorrichtung nach einem der Ansprüche 1 bis 7, **dadurch gekennzeichnet, dass** das Oberteil (4) eine Basisplatte (4.4) und einen die Anzuchtbehälter (4.1) tragenden Aufsatz (4.5) aufweist, wobei der Aufsatz (4.5) und die Basisplatte (4.4) in der Gebrauchslage der Probennahme-Vorrichtung miteinander lösbar verbunden sind.

9. Probennahme-Vorrichtung nach Anspruch 8, **dadurch gekennzeichnet, dass** die Bodenöffnung (4.1.1) jedes Anzuchtbehälters (4.1) des Aufsatzes (4.5) außenseitig mit einem rohrartigen Kragen (4.6) umgeben ist, wobei der Kragen (4.6) in der Gebrauchslage der Probennahme-Vorrichtung im Wesentlichen bis an das dem Aufsatz (4.5) abgewandte Ende der Basisplatte (4.4) reicht.

10. Probennahme-Vorrichtung nach einem der Ansprüche 1 bis 9, **dadurch gekennzeichnet, dass** mindestens zwei Bauteile aus der Gruppe Oberteil (4), Basisplatte (4.4), Aufsatz (4.5), Unterteil (2), Schneidplatte (8) und Messer (6) zueinander korrespondierende Positioniermittel (4.6, 4.4.1) aufweisen.

11. Probennahme-Vorrichtung nach einem der Ansprüche 1 bis 10, **dadurch gekennzeichnet, dass** mindestens einzelne Komponenten des Unterteils (2) und/oder des Oberteils (4) und/oder das Messer (6) und/oder die Klemmen (12, 14) aus einem Kunststoff hergestellt sind und dass bevorzugt die Probebehälter (2.1) und/oder die Aufzuchtbehälter (4.1) und/oder die Basisplatte (4.4) aus Kunststoff hergestellt sind
